(19) Europäisches Patentamt
European Patent Office
Office européen des brevets

(11) Veröffentlichungsnummer: **0 206 054**
**B1**

(12) # EUROPÄISCHE PATENTSCHRIFT

(45) Veröffentlichungstag der Patentschrift: **18.10.89**

(51) Int. Cl.⁴: **C 07 C 51/235**, C 07 C 53/00

(21) Anmeldenummer: **86107732.9**

(22) Anmeldetag: **06.06.86**

(54) **Verfahren zur Herstellung von Carbonsäuren.**

(30) Priorität: **20.06.85 DE 3522032**

(43) Veröffentlichungstag der Anmeldung:
**30.12.86 Patentblatt 86/52**

(45) Bekanntmachung des Hinweises auf die Patenterteilung:
**18.10.89 Patentblatt 89/42**

(84) Benannte Vertragsstaaten:
**BE CH DE FR GB IT LI NL**

(56) Entgegenhaltungen:
**DE-A- 3 135 946**
**GB-A- 2 010 248**
**US-A- 3 407 220**
**US-A- 4 238 625**

(73) Patentinhaber: **HOECHST AKTIENGESELLSCHAFT, Postfach 80 03 20, D-6230 Frankfurt am Main 80 (DE)**

(72) Erfinder: **Leupold, Ernst Ingo, Dr., Am Zäunefeld 15, D-6392 Neu-Anspach (DE)**

**EP 0 206 054 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein Verfahren zur Herstellung von Carbonsäuren durch platinkatalysierte Oxidation von begrenzt wasserlöslichen primären Alkoholen mit Sauerstoff in einem Gemisch aus Wasser und einem Lösevermittler.

Die Herstellung von Carbonsäuren durch Oxidation von primären Alkoholen mit Sauerstoff in Gegenwart von Platinkatalysatoren ist seit längerem bekannt. Im allgemeinen wird die Reaktion in Wasser als Lösemittel durchgeführt (US-PS 3 342 858, US-PS 3 799 977, DE-OS 2 936 123).

Bei begrenzter Wasserlöslichkeit von Alkohol und Carbonsäure werden Kohlenwasserstoffe als Lösemittel verwendet (US-PS 3 407 229). Dies ist jedoch mit mehreren Nachteilen verbunden. So läuft die Reaktion nur unter erhöhtem Sauerstoffpartialdruck mit vertretbar hoher Reaktionsgeschwindigkeit ab; weiterhin erfordert die Verwendung von Kohlenwasserstoffen in Gegenwart von Sauerstoff aufwendige Sicherheitsmaßnahmen, um das Risiko einer Explosion gering zu halten.

Man kann auch in Wasser arbeiten, wenn man die gebildete Carbonsäure zunächst umgehend in das entsprechende Alkalisalz überführt (US-PS 4 238 625). Der dafür notwendige Einsatz von Basen in molaren Mengen führt aber bei der anschließenden Freisetzung der gewünschten Carbonsäure mit einer anorganischen Säure zur Bildung von molaren Salzmengen, deren Entsorgung mit erheblichen Kosten verbunden ist.

Es wurden bereits vorgeschlagen, begrenzt wasserlösliche primäre Alkohole in Gegenwart eines Lösevermittlers in Wasser an Platinkatalysatoren zu oxidieren, jedoch wurden keine konkreten Angaben über die Art einer solchen Komponente gemacht (DE-OS 2 851 788).

Somit bestand die Aufgabe, einen geeigneten Lösevermittler für die Oxidation von begrenzt wasserlöslichen primären Alkoholen in Wasser mit Sauerstoff an Platinkatalysatoren zu ermitteln.

Ein geeigneter Lösevermittler sollte mehrere Eigenschaften aufweisen:

— inert unter den Reaktionsbedingungen

— nicht flüchtig mit überschüssigem Sauerstoff bei den üblichen Reaktionstemperaturen von 35 bis 95°C, um die Explosionsgefahr im Dampfraum zu vermeiden

— leicht abtrennbar aus dem Reaktionsgemisch.

Diese für eine wirfschaftliche Nutzung notwendigen Eigenschaften lassen übliche Lösevermittler, wie tert.-Butanol, Aceton, Dioxan, Tetrahydrofuran, als ungeeignet für den genannten Zweck erscheinen.

Gegenstand der vorliegenden Erfindung ist daher ein Verfahren zur Herstellung von Carbonsäuren durch platinkatalysierte Oxidation von begrenzt wasserlöslichen primären Alkoholen mit Sauerstoff in einem Gemisch aus Wasser und einem Lösevermittler, dadurch gekennzeichnet, daß man als Lösevermittler Ether der allgemeinen Formel

$$R_1O(CH_2CH_2O)_nR_2$$

einsetzt, wobei $n = 1\text{-}4$ ist und $R_1$ und $R_2$ Alkylreste mit 1 bis 4 Kohlenstoffatomen sind.

Nach dem erfindungsgemäßen Verfahren können nun auch in Wasser schwer lösliche primäre Alkohole in einfacher und wirtschaftlicher Weise oxidiert werden. Dabei ist die oben erwähnte Verwendung von Alkalihydroxid unnötig, so daß bei der Aufarbeitung die teure Entsorgung anorganischer Salze entfällt. Gegenüber der Verwendung eines Kohlenwasserstoffs als Lösemittel oder der Verwendung eines unter den Reaktionsbedingungen flüchtigen Lösevermittlers ist das erfindungsgemäße Verfahren technisch erheblich einfacher und sicherer durchführbar, da im Dampfraum neben Sauerstoff hauptsächlich Wasser vorhanden ist und damit explosive Gasgemische praktisch vermieden werden.

Die als Lösevermittler eingesetzten Glykolether haben die allgemeine Formel $R_1O(CH_2CH_2O)_nR_2$, wobei $n = 1\text{-}4$ ist und $R_1$ und $R_2$ Alkylreste mit 1-4 C-Atomen sind. Bevorzugt unter diesen sind die Dimethyl-, Diethyl- oder Methyl-ethylether der Formeln $CH_3O(CH_2CH_2O)_nCH_3$, $C_2H_5O(CH_2CH_2O)_nC_2H_5$, bzw. $CH_3O(CH_2CH_2O)_nC_2H_5$, vor allem die Dimethylether. Gemessen an den Kriterien: inert, geringe Flüchtigkeit und leichte Abtrennbarkeit sind Glykolether mit Siedepunkten im Bereich von 100 bis etwa 250°C, wie Diethylenglykoldimethylether und Triethylenglykoldimethylether besonders geeignet.

Es können auch Glykolether der allgemeinen Formel $R_1O(CH_2CH_2O)_nR_2$ mit $n > 4$ und/oder mit Alkylresten $R_1$, $R_2$ mit mehr als 4 Kohlenstoffatomen eingesetzt werden; jedoch ist der Aufwand für die destillative Rückgewinnung wegen der relativ hohen Siedepunkte größer.

Es können aber auch andere Ether, wie z.B. Propylenglykolether oder Kronenether, als Lösevermittler verwendet werden, wenn hinsichtlich Verfügbarkeit und Preis eine wirtschaftliche Nutzung des Verfahrens gewährleistet ist.

Die Menge des eingesetzten Ethers in Relation zur Wassermenge kann in weiten Grenzen schwanken. Zweckmäßig wählt man die Menge so, daß der eingesetzte Alkohol bei der Reaktionstemperatur gerade vollständig gelöst ist. Dies bedeutet im allgemeinen ein Verhältnis Wasser/Lösevermittler zwischen 0,1 und 100.

Prinzipiell können nach dem erfindungsgemäßen Verfahren praktisch alle in Wasser begrenzt löslichen primären Alkohole zu den entsprechenden Carbonsäuren oxidiert werden, also Alkohole, die n- und/oder iso-Alkyl- und/oder Cycloalkyl- und/oder Aryl-Substituenten tragen, wobei auch zusätzliche Heteroatome vorhanden sein können.

Es können beispielsweise eingesetzt werden: aliphatische Alkohole, wie 1-Pentanol, 2-Methyl-1-butanol, 3-Methyl-1-butanol, 2.2-Dimethyl-1-propanol, 1-Hexanol, 2-Methyl-1-pentanol, 2-Ethyl-1-butanol, 1-Heptanol, 1-Octanol, 2-Ethyl-1-hexanol, Isooctanol, 1-Nonanol, 1-Decanol, 1-Undecanol, 1-Dodecanol, 1-Tridecanol, 1-Tetradecanol, 1-Pentadecanol, 1-Hexadecanol, 1-Heptadecanol, 1-Octadecanol, 1-Nonadecanol, 1-Eicosanol, 1-Docosanol, 1-Tetracosanol, 1-Hexacosanol, 1-Octacosanol; oder Glykolether der allgemeinen Formel $RO(CH_2CH_2O)_nH$ mit R = Alkyl-, Cyclo-alkyl- oder Aryl- und $n \geqslant 1$, wie Butylglykol, Pentylglykol, Hexylglykol, Cyclohexylglykol, 2-Ethylhexylglykol,

Phenylglykol, o-sek.-Butylphenylglykol, p-Nonylphenylglykol, Hexyldiglykol, 2-Ethylhexyltriglykol, Phenylpolyglykol.

Vorzugsweise werden die Alkohole in Form einer 10 bis 50%igen Lösung in dem Gemisch aus Wasser und Lösevermittler eingesetzt. Niedrigere Konzentrationen sind prinzipiell möglich, jedoch steigt damit der Aufwand für die Isolierung des Reaktionsproduktes. Höhere Konzentrationen sind ebenfalls möglich; allerdings nimmt die Reaktionsgeschwindigkeit mit steigender Alkoholkonzentration allmählich ab.

Die Reaktionstemperatur liegt im allgemeinen zwischen 20 und 150°C, wobei ggf. Überdruck angewandt werden muß, um diese Temperatur zu erreichen. Bevorzugt ist der Bereich von 35 bis 95°C, weil dann eine besonders hohe Selektivität und Reaktivität erzielt wird.

Bevorzugtes Oxidationsmittel ist reiner Sauerstoff; es können jedoch auch Mischungen von Sauerstoff mit Inertgasen wie Luft, verwendet werden.

Als Platinkatalysatoren eignen sich handelsübliche Trägerkatalysatoren, insbesondere Aktivkohlen mit 5 bis 10 Gew.-% Platin.

Die Anwendung von Druck ist nicht zwingend erforderlich, jedoch steigt die Reaktionsgeschwindigkeit mit dem Sauerstoffpartialdruck deutlich an. Bevorzugt ist daher ein Druckbereich von 1 bis 10 bar (absolut). Bei höherem Druck z.B. 100 bar, läuft die Reaktion noch schneller ab; allerdings kann dann der Vorteil der höheren Reaktionsgeschwindigkeit durch höhere Investitionen kompensiert werden.

Das erfindungsgemäße Verfahren kann in allen Apparaturen, die sich für die Durchführung von Reaktionen in der Flüssigphase mit oder ohne Anwendung von Überdruck eignen, durchgeführt werden, beispielsweise in einem Rührkessel oder in einer Blasensäule mit suspendiertem Katalysator; es können aber auch Festbettreaktoren mit gekörntem Katalysator als Rieselphasenreaktoren eingesetzt werden.

Das Reaktionsgemisch kann nach bekannten Methoden aufgearbeitet werden. Zweckmäßig ist eine Destillation, wobei Wasser und Lösevermittler sowie nicht umgesetztes Ausgangsprodukt im allgemeinen zuerst übergehen und in den Reaktor zurückgeführt werden können. Je nach den Reinheitsanforderungen kann die Carbonsäure nun entweder direkt oder nach einer destillativen Reinigung ihrer weiteren Verwendung zugeführt werden.

*Beispiel 1*

In ein von außen beheiztes senkrecht angeordnetes Glasrohr (Durchmesser: 50 mm, Länge: 800 mm), das mit einer Mischung aus 200 g n-Octanol-(1), 175 g Wasser, 550 g Diglykoldimethylether und 50 g eines handelsüblichen Katalysators (5% Platin auf Aktivkohle) gefüllt war, leitete man von unten durch eine Glasfritte 25 Nl/h Sauerstoff bei einer Temperatur von 90°C. Die Reaktionszeit betrug 20 h. Die filtrierte Reaktionslösung enthielt 19,7 Gew.-% Caprylsäure. Bei der destillativen Aufarbeitung erhielt man nach Abtrennung von Wasser und Diglykoldimethylether 171,4 g Caprylsäure, entsprechend einer Ausbeute von 77,4%.

*Vergleichsbeispiel*

Man ersetzte den Lösevermittler durch die gleiche Menge Wasser und führte den versuch unter sonst identischen Bedingungen, wie in Beispiel 1 beschrieben, durch. Das Reaktionsgemisch enthielt weniger als 1 Gew.-% Caprylsäure.

*Beispiel 2*

Unter den in Beispiel 1 beschriebenen Bedingungen wurde ein Gemisch von 200 g 2-Phenoxyethanol, 500 g Wasser, 300 g Diglykoldimethylether und 50 g Katalysator 22 h bei 80°C mit Sauerstoff umgesetzt. Die filtrierte Reaktionslösung enthielt 14,4 Gew.-% 2-Phenoxyessigsäure.

*Beispiel 3*

Unter den in Beispiel 1 angegebenen Bedingungen wurden 200 g ortho-sek.-Butylphenoxyethanol in 255 g Wasser und 545 g Diglykoldimethylether in Gegenwart von 50 g Katalysator bei 80°C 23 h mit Sauerstoff oxidiert. In der filtrierten Reaktionslösung wurden 16,3 Gew.-% ortho-sek.-Butylphenoxyessigsäure nachgewiesen.

*Beispiel 4*

Wie in Beispiel 1 beschrieben, wurde eine Mischung aus 200 g n-Octanol-(1), 250 g Wasser, 550 g Tetraethylenglykol-methyl-tert.-butylether und 40 g Katalysator 25 h bei 80°C mit Sauerstoff oxidiert. Die filtrierte Reaktionslösung enthielt 18,3 Gew.-% Caprylsäure.

*Beispiel 5*

Analog Beispiel 1 wurde ein Gemisch aus 200 g 2-Ethylhexanol, 650 g Diglykoldimethylether, 150 g Wasser und 50 g Katalysator 14 h bei 90°C mit Sauerstoff umgesetzt. Die filtrierte Reaktionslösung enthielt 18,3 Gew.-% 2-Ethylhexansäure.

*Beispiel 6*

Wie in Beispiel 1 beschrieben, wurden 200 g 2-Ethylhexylglykol, 550 g Diglykoldimethylether, 250 g Wasser und 50 g Katalysator 6 h bei 80°C mit Sauerstoff umgesetzt:

$$CH_3(CH_2)_3CH(C_2H_5)CH_2OCH_2CH_2OH \rightarrow$$

$$\rightarrow CH_3(CH_2)_3CH(C_2H_5)CH_2OCH_2COOH$$

Die filtrierte Reaktionslösung enthielt 20,6 Gew.-% 2-Ethylhexoxyessigsäure. Bei der destillativen Aufarbeitung erhielt man nach Abtrennung von Wasser und Diglykoldimethylether 163 g reine 2-Ethylhexoxyessigsäure mit einem Siedepunkt von 115°C bei 3 mbar, entsprechend einer Reinausbeute von 75,4%.

*Beispiel 7*

Analog Beispiel 1 wurden 200 g eines Gemisches isomerer p-iso-Nonylphenoxyethanole (mit unterschiedlich verzweigtem Nonylrest), 600 g Triglykoldimethylether, 200 g Wasser und 50 g Katalysator 11 h bei 90°C mit Sauerstoff umgesetzt:

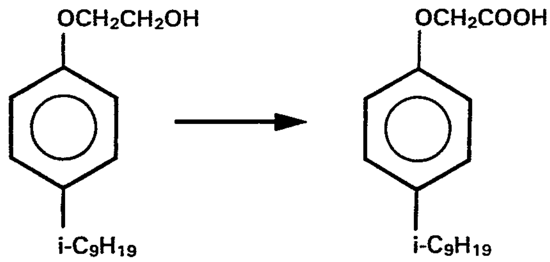

Nach Abtrennung des Katalysator enthielt die Reaktionslösung 19,2 Gew.-% isomerer p-iso-Nonylphenoxyessigsäuren.

*Beispiele 8-10*

In ein von außen beheiztes senkrecht angeordnetes Glasrohr (Durchmesser: 25 mm, Länge 200 mm), das mit einer Mischung aus 35 ml Diglykoldimethylether, 5 ml $H_2O$, 2,5 g eines Katalysators (5% Pt auf Aktivkohle) und 10 g eines Isomerengemischs von Alkoholen gleicher Kohlenstoffzahl mit primären Hydroxylgruppen, wie man es z.B. aus der Hydroformylierung von Olefingemischen und anschließender Hydrierung erhält, gefüllt war, leitete man von unten durch eine Glasfritte 10 Nl/h Sauerstoff bei 80°C über einen Zeitraum von 12 h. Die filtrierte Reaktionslösung enthielt die in der Tabelle angegebenen Mengen an Carbonsäureisomeren:

TABELLE

| Beispiel | Alkohol | Carbonsäure | Gehalt [Gew.-%] |
|---|---|---|---|
| 8 | Isotridecanol | Isotridecansäure | 18.1 |
| 9 | Isohexadecanol | Isohexadecansäure | 18.6 |
| 10 | Isooctadecanol | Isooctadecansäure | 19.8 |

*Beispiel 11*

Analog den Beispielen 8-10 wurden 10 g 8-Hydroxymethyl-tricyclo[5.2.1.$0^{2.6}$]decan in Gegenwart von 30 g Diglykol-dimethylether, 10 g Wasser und 2,5 g Katalysator 14 h bei 80°C mit Sauerstoff behandelt. Die filtrierte Reaktionslösung enthielt 19,1 Gew.-% 8-Carboxytricyclo[5.2.1.$0^{2.6}$]decan.

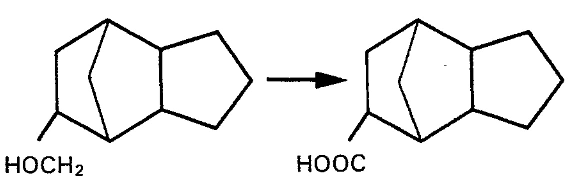

*Beispiel 12*

Analog den Beispielen 8-11 wurden 10 g eines Gemischs aus p-iso-Nonylphenylpolyglykolen der allgemeinen Formel

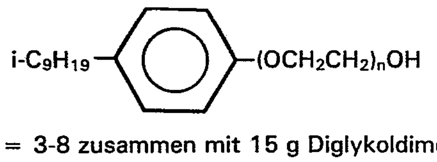

mit n = 3-8 zusammen mit 15 g Diglykoldimethylether, 25 g Wasser und 2,5 g Katalysator 10 h bei 60°C mit Sauerstoff umgesetzt. Die filtrierte Reaktionslösung enthielt 18,3 Gew.-% eines Gemischs der entsprechenden p-iso-Nonylphenylpolyglykolsäuren.

## Patentansprüche

1. Verfahren zur Herstellung von Carbonsäuren durch platinkatalysierte Oxidation von begrenzt wasserlöslichen primären Alkoholen mit Sauerstoff in einem Gemisch aus Wasser und einem Lösevermittler, dadurch gekennzeichnet, daß man als Lösevermittler Ether der allgemeinen Formel

$$R_1O(CH_2CH_2O)_nR_2$$

einsetzt, wobei n = 1-4 ist und $R_1$ und $R_2$ Alkylreste mit 1-4 Kohlenstoffatomen sind.

2. Verfahren nach Anspruch 1, dadurch gekennzeichnet, daß Diethylenglykoldimethylether oder Triethylenglykoldimethylether als Lösevermittler eingesetzt wird.

3. Verfahren nach einem der Ansprüche 1 oder 2, dadurch gekennzeichnet, daß das Verhältnis Wasser/Lösevermittler zwischen 0,1 und 100 liegt.

## Claims

1. A process for preparing carboxylic acids by platinum-catalyzed oxidation of primary alcohols of limited water solubility with oxygen in a mixture of water and a solubilizer, which comprises using as the solubilizer ethers of the formula

$$R_1O(CH_2CH_2O)_nR_2,$$

where n is 1-4 and $R_1$ and $R_2$ are alkyl radicals having 1-4 carbon atoms.

2. The process as claimed in claim 1, wherein diethylene glycol dimethyl ether or triethylene glycol dimethyl ether is used as the solubilizer.

3. The process as claimed in either of claims 1 or 2, wherein the water/solubilizer ratio is between 0.1 and 100.

## Revendications

1. Procédé pour préparer des acides carboxyliques par oxydation, catalysée par du platine, d'alcools primaires, à solubilité limitée dans l'eau, avec de l'oxygène dans un mélange d'eau et d'un tiers-solvant, procédé caractérisé en ce qu'on utilise comme tiers-solvants des éthers répondant à la formule générale

$R_1O(CH_2CH_2O)_nR_2$,

dans laquelle n vaut 1 à 4 et $R_1$ et $R_2$ représentent des restes alkyles comportant 1 à 4 atomes de carbone.

2. Procédé selon la revendication 1, caractérisé en ce qu'on utilise comme tiers-solvant de l'éther-oxyde diméthylique de diéthylène-glycol ou de l'éther-oxyde diméthylique de triéthylène-glycol.

3. Procédé selon l'une des revendications 1 ou 2, caractérisé en ce que le rapport eau/tiers-solvant se situe entre 0,1 et 100.